# EUROPEAN PATENT SPECIFICATION

(11) **EP 2 212 307 B1**
(45) Date of publication and mention of the grant of the patent: **28.11.2012**
(21) Application number: 08842543.4
(22) Date of filing: 22.10.2008
(51) Int. Cl.: C07D 307/92

(54) **HIGH PURITY SYNTHETIC PROCESS FOR THE PREPARATION OF DODECAHYDRO-NAPTHO-FURANYL-CARBAMIC ACID ESTER INTERMEDIATES**
HOCHREINES SYNTHETISCHES VERFAHREN ZUR HERSTELLUNG VON DODECAHYDRONAPHTHOFURANYLCARBAMINSÄUREESTERZWISCHENPRODUKTEN
PROCÉDÉ DE SYNTHÈSE HAUTE PURETÉ POUR LA PRÉPARATION DE PRODUITS INTERMÉDIAIRES D'ESTER D'ACIDE DODÉCAHYDRO-NAPHTO-FURANYL-CARBAMIQUE

(30) Priority: 24.10.2007 US 164
(43) Date of publication of application: 04.08.2010
(73) Proprietor: Merck Sharp & Dohme Corp., Rahway, NJ 07065-0907 (US)
(72) Inventor: LIAO, Jing, Livingston New Jersey 07039 (US); WANG, Tao, Springfield NewJersey 07081 (US)
(74) Representative: Hussain, Deeba
(86) International application number: PCT/US2008/080699
(87) International publication number: WO 2009/055416

(56) References cited:
- WO-A-2006/076452
- US-A1- 2006 247 450

## Description

### Field of the Invention

This application discloses a novel process to prepare furanyl carbamic acid ester compounds of Formula Ia in high purity, which have utility, for example, as intermediates in the preparation of pharmaceutically active himbacine analog compounds.

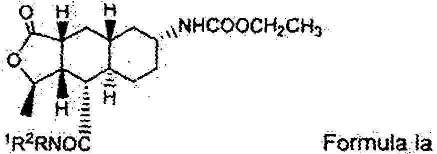

### Background of the Invention

Identification of any publication in this section or any section of this application is not an admission that such publication is prior art to the present invention.

U.S. Patent No. 6,063,847, describes himbacine analogs known to act as thrombin receptor antagonists which have useful pharmaceutical activity, for example, the compound of Formula II.

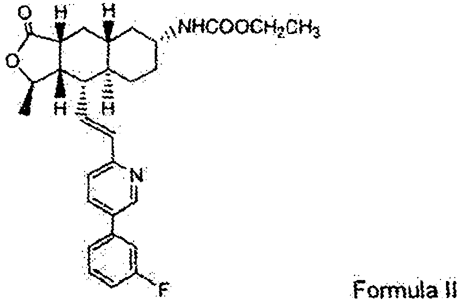

In some preparation schemes a compound of Formula Ia, for example, the compound of Formula I, 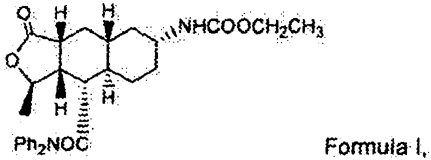 is a critical intermediate in the preparation of himbacine analog compounds having desirable pharmaceutical properties, for example, the compound of Formula II. U.S. Patent Application Serial No. 11/331,324, filed January 12, 2006 and published under Publication No. 2006/0247450 (the 450 publication), describes in Example 7 the preparation of the compound of Formula II from the compound of Formula I. Preparation of the intermediate compounds of Formula Ia from commercially available starting materials has been accomplished using a multi-step synthesis, for example, as described in the '450 publication, page 13, paragraph [0074] to page 16, paragraph [0078]. For Example, the '450 publication describes on page 8, the preparation of compounds of Formula Ia in accordance with Scheme I,

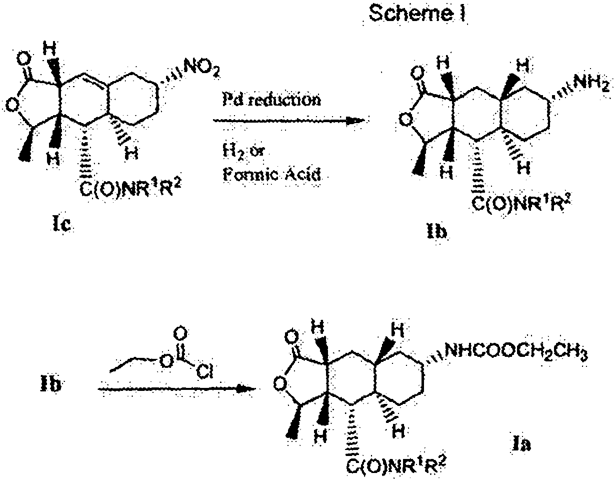

The purity of compounds of Formula Ia provided by the procedure described in the '450 publication is highly sensitive to the purity of the precursor compound of Formula Ic (referencing Scheme I) used in the preparative process. For an example, see the '450 publication at Example 7, starting at paragraph [0098]. When synthesized in accordance with the '450 publication, the product comprising the compound of Formula I requires a number of recrystallizations to consistently provide the compound of Formula I having suitable purity for use in the synthesis of compounds of Formula II. Without purification, the yield and handling properties of the target compound are greatly reduced.

WO 2006/076452 also discloses a process for the preparation of himbacine analoges. More particularly, the compound of formula I is obtained by hydrogenation of double blond of a carbamic acid followed by purification by column chromatography.

### Objectives and Summary of the Invention

In view of the foregoing, what is needed is method for preparing consistently high purity quantities of the intermediate compounds of Formula Ia, for example, the compound of Formula **I.** Moreover, what is needed is a process for providing compounds of Formula Ia in which product purity is less sensitive to the purity of precursor materials and which minimizes the need for recrystallization steps to consistently provide compounds of Formula Ia in high purity. What is needed also is a process for providing the compound of Formula I that can be adapted to a batch size suitable for commercial scale preparation. These and other objectives and/or advantages are provided by the present invention.

One preferred aspect of the present invention is a process for providing a purified form of the compound of Formula Ia, 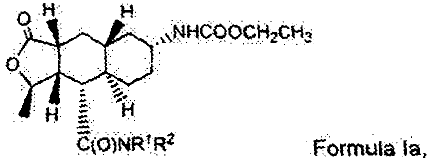 the process comprising:
(a) providing a saturated solution comprising the compound of Formula I in a solvent comprising substantially acetone or 2-methyl-tetrahydrofuran (2-Me-THF), at a first temperature, wherein the first temperature is above ambient and is selected to be in a range suitable to form a suspension comprising crystals of the compound of Formula I and maintaining the solution at said first temperature for a period of time sufficient to form a suspension of the compound of Formula **I,** optionally while agitating the mixture;
(b) optionally reducing the temperature of the suspension formed in Step "a" to a second temperature, wherein said second temperature is subambient;
(c) optionally agitating the suspension from step 'b" while holding it at said second temperature for a period sufficient to precipitate a crystalline product having less than 3.0 mole%, preferably less than 2.0 mole% impurity and comprising at least 80% of the compound of Formula I initially present in the solution provided in Step "a"; and
(d) optionally isolating the crystalline product precipitated in Step "c"

In some embodiments, it is preferred to provide the purified compound of Formula Ia in the form of a solvate. In some embodiments It is preferred to select in step "b" a second temperature of from -5°C to +10°C. In some embodiments it is preferred to reduce the temperature of the suspension in step "b" from said first temperature to said second temperature over a period of up to 3 hours, preferably from 1 to 3 hours. In some embodiments employing optional step "c° it is preferred to carry out the agitation over a period of from 1 to 2 hours. In some embodiments it is preferred after carrying out optional step "d" to dry the isolated precipitate at a temperature proximal to ambient so as to provide the isolated precipitate in the form of a solvate.

In some embodiments it is preferred to carry out this process to provide a purified form of the compound of Formula I using acetone as a purifying solvent, thereby providing a crystalline acetone monosolvate form of the compound of Formula I wherein the crystalline monosolvate product provides an NMR spectrum in deuterochloroform having the following features, ¹HNMR (CDCl₃) δ 7 25 - 7 55 (m, 10 H), 4.89(m, 1H). 4.51 (bs, 1H) 4.09 (d, J = 6.98 Hz, 2H). 3.49 (brs, 1H). 2.41 (m 2H), 2.25 (m, 1H), 2 19(s, 6 H Acetone) 2 06 (d, J = 10.8 Hz, 2H), 1.96 (d J = 10.9 Hz, 1H), 1 83 (ddd J=13.5.6 09. 2 51 Hz, 1H), 1 63(m, 1H), 1.52 (d, J =5.8 Hz, 3H). 1 23 (m, 5H), 1.17 (q, J = 11.5Hz, 2H) 0 92 (q J = 11.5 Hz 1H)

In some embodiments it is preferred to carry out this process to provide a purified form of the compound of Formula I using 2-methyl-tetrahydrofuran (2-Me-THF) as a purifying solvent, thereby providing a crystalline 2-Me-THF monosolvate form of the compound of Formula I wherein the crystalline monosolvate product provides an NMR spectrum in deuterochloroform having the following features ¹HNMR (CDCl₃) δ 7.25 - 7.55 (m, 10 H), 4.89(m, 1H), 4.51 (bs, 1H), 4.09 (d, J = 6.98 Hz, 2H), 3.98-3.86 ( m, 2H, 2-Me-THF), 3.73-3.68 (m, 1H, 2-Me-THF), 3.49 (brs, 1H), 2.41 (m, 2H), 2.25 ( m, 1H), 2.06 (d, J = 10.8 Hz, 2H), 1.96 (d, J = 10.9 Hz, 1H), 1.92-1.79(m, 3H, 2-Me-THF),1.83 (ddd, J = 13.5, 6.09, 2.51 Hz, 1H), 1.63(m, 1H), 1.52 (d, J = 5.8 Hz, 3H), 1.45-1.36( m, 1H, 2-methyl-THF) 1.23 (m, 5H), 1.22 (d, 3H, 2-Me-THF) 1.17 (q, J = 11.5 Hz, 2H), 0.92 (q, J = 11.5 Hz, 1H).

In one embodiment, the precipitation process is incorporated into a process for synthesis of the compound of Formula la in accordance with Scheme II, 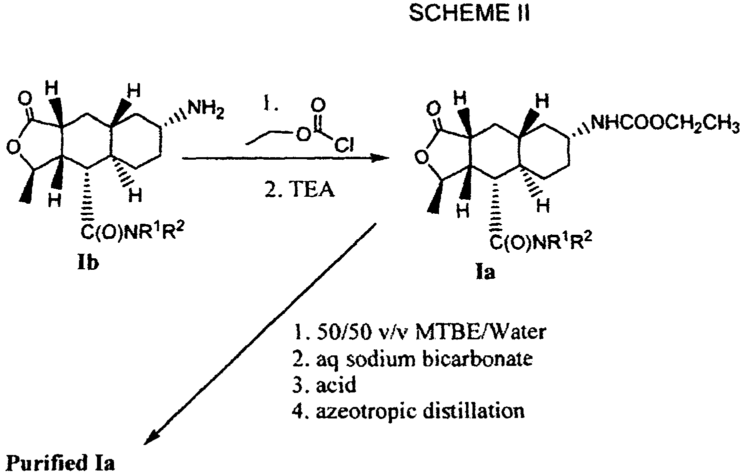 the process comprising;
(i) providing a THF solution of the compound of Formula Ib;
(ii) forming the amide of Formula Ia in a reaction mixture provided by treating the THF solution from step "i" first with ethylchloroformate and adding thereafter triethyl amine;
(iii) working up the reaction mixture from step "ii" by adding equal volumes of water and methyl tert-butyl ether (MTBE) and then separating the resulting organic and aqueous phases;
(iv) mixing the separated organic phase from step "iii" with an equal volume of water and an aliquot of a carbonate base solution:
(v) adjusting the pH of the mixture provided In Step "iv" with an aqueous acid to a pH of from pH 6 to pH 7.5 and separating the aqueous and organic phases of the mixture;
(vi) modifying the solvent in the separated organic phase obtained in Step "v" by a process comprising:
   a) mixing a purifying solvent with the separated organic phase obtained in Step "v", wherein the purifying solvent is acetone or 2-Me-THF,
   b) azetropically distilling the mixture until it is concentrated; and
   c) repealing steps "a" and "b" until the solvent in the mixture is substantially the purifying solvent; and
(vii) cooling the phase provided in Step "vii", thereby precipitating a substantially pure solvate form of the compound of Formula Ia.

In some embodiments of the process it is preferred for the compound of Formula Ia to be the compounds of Formula I, thus, R¹ and R² in each occurrence in the compounds of Formulae Ic, Ib, and Ia is phenyl.

In some embodiments it is preferred to provide the compound of Formula Ib by hydrogenating the compound of Formula Ic, 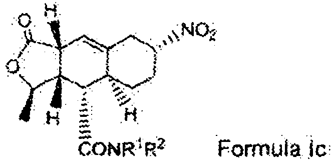 using a palladium catalyst and a mixed water/THF reaction medium.

In some embodiments of the inventive process it is preferred in Step "iv" to employ an aqueous 5% sodium bicarbonate solution as the carbonate base solution. In some embodiments of the inventive process it is preferred to employ in Step "v" an aqueous sulfuric acid solution to adjust the pH of the mixture In some embodiments of the inventive process it is preferred to concentrate the separated organic phase by distilling off solvent prior to carrying out solvent modification in Step "vi"

in some embodiments of the inventive process. In the synthesis process of Scheme I, step "vii" comprises holding a saturated solution of the compound of Formula Ia at a first temperature, wherein, nucleation of crystals of the compound of Formula Ia are formed, producing a suspension of the compound of Formula Ia, followed by a reduction in temperature to a second temperature at which a crystalline form, preferably a crystalline solvate form, of the compound of Formula Ia is precipitated from the modified organic phase, preferably holding the phase at a second temperature, preferable a subambient temperature, at which precipitation of a crystalline form of the compound of Formula Ia is maximized. In some embodiments wherein the solvate form of the compound of Formula Ia precipitated in Step "vii" is a 2-methyl-tetrahydrofuran mono-solvate form or an acetone mono-solvate form of the compound of Formula I, preferably said first temperature is a temperature of from 45 °C to 60 °C, and said second temperature is preferably a temperature of from -5 °C to +10 °C.

These and other aspects of the invention are further described in the following description.

### Detailed Description of the Invention

A process for providing the compound of Formula I is described in the '450 publication in Example 7. With reference to Scheme i, above, the '450 publication describes preparing the compound of Formula Ib from the compound of Formula Ic (wherein, in each instance, R¹ and R² are each phenyl) by hydrogenation over palladium catalyst, and subsequently reacting the amine intermediate thereby formed with a chloroformate to provide the carbamic acid ester compound of Formula I. As mentioned above, the process described in the '450 publication provides high impurity levels in the intermediate compound of Formula I when the chloroformate reaction is worked up with subsequent precipitation of the compound of Formula I from the mixture resulting from treating the reaction mixture with an ethanol/water mixture followed by azeotropic distillation of the mixture in accordance with the teaching of Example 7 in the '450 publication.

Attempts to use various solvents, for example, Methyl-tert.-Butyl Ether (MTBE); and various solvent/antisolvent systems, for example, ethylacetate/heptane and toluene/heptane, to recrystallize aliquots of the pricipiate comprising the compound of Formula I made in accordance with the process described in the '450 publication resulted in the formation of oils. These oils did not yield a reduced impurity profile in synthesis of the compound of Formula I, nor provide an improvement in the quality of the product compound of Formula II provided from the process described in the '450 publication.

Accordingly, in one aspect, the process of the present invention provides improvements in the synthetic process as described in the '450 publication by adapting the present invention to that process to reduce the level of impurities present in the precipitation product comprising the compound of Formula la made in accordance therewith.

The inventors have surprisingly found that the present invention process, which mixes a reaction mixture containing a compound of Formula la (after workup) with a purifying solvent, and after subsequent concentration and volatiles stripping, precipitates therefrom either a monoacetone solvate form or a mono-2-methyl-tetrahydrofuran solvate form of a compound of Formula la, for example, the monoacetone solvate form of the compound of Formula I, providing the compound of Formula la in a form containing substantially lower percentages of impurities present in the precipitated material in comparison with the precipitated material comprising the compound of Formule Ia obtained from mixing the reaction mixture workup with ethanol/water solvent in accordance with the process described in the '450 publication.

When the process of the present invention is part of a reaction scheme for preparing a compound of Formula Ia, preferably, the process of providing a compound of Formula Ia is carried out in accordance with the methodology described in the above-referenced '450 publication (see Example 7 therein) up through the step of extracting the product in the reaction mixture into methyl-tertiarybutyl ether (MTBE) In accordance with the present invention, the extract thus obtained which contains the compound of Formula la is then mixed with a purifying solvent, for example, 2-methyl-tetrahydrofuran or acetone, and concentrated by distilling off the mixture, azeotropically, until the solvent remaining in the concentrate substantially comprises the purifying solvent. The temperature of the concentrate thus provided is then reduced to a temperature (first crystallization temperature) at witch formation of a crystalline solvate form of the compound of Formula Ia commences, for example, the crystalline monoacetone solvate of the compound of Formula I, forming a crystalline suspension or precipitation product comprising the desired crystalline mono-solvate product of the compound of Formula Ia. In some embodiments of the process providing the monoacetone solvate form of the compound of Formula I, it is preferred to hold the concentrate at a first crystallization temperature of from 45 °C to 60°C.

In some embodiments wherein the compound of Formula Ia is purified using either acetone or 2-methyl-tetrahydrofuran as the purifying solvent, after holding the concentrate at a first crystallization temperature for a period of time sufficient to commence precipitation of the compound of Formula Ia in a monoacetone solvate crystalline form or tne 2-methyl-tetrahydrofuran crystalline form, respectively, the temperature of the solution is lowered to a subambient temperature in a controlled manger, wherein the cooling rate and the final temperature are selected to maximize the amount of the crystalline solvate form of the compound precipitated from the concentrate while preserving in the precipitated crystalline material the low level of impurities provided by the precipitation process. Preferably, when the compound of Formula Ia is the compound of Formula I, the first crystallization temperature is a temperature of from 45 °C to 60 °C, and the second crystallization temperature is a temperature of from 5 °C to +10 °C, although it will be appreciated that temperature values lying outside of these ranges can be employed without departing from the process of the present invention. In some embodiments it is preferred to maintain the first crystallization temperature for a period of up to 1 hour, more preferably 1 hour, although longer periods may also be employed without departing from the process of the present invention. In some embodiments it is preferred to lower the temperature from the first crystallization temperature to the second crytallization temperature over a period of 3 hours, although faster and slower cooling rates may also be employed without departing from the process of the present invention. In some embodiments it is preferred to hold the second crystallization temperature for a period of from 1 to 2 hours, although it will be appreciated that longer and shorter holding periods may be employed without departing from the process of the present invention. In some embodiments it will be appreciated that the slurry may be agitated for some or all of the second crystallization temperature holding period.

Although it will be appreciated that the advantages of the present invention can be realized utilizing aliquots of a compound of Formula Ia prepared by any means, and dissolving the aliquot of the compound in a purifying solvent, for example, acetone and 2-methy-tetrahydrofuran from which it is reprecipitated as the mono-solvate crystalline form of the compound of Formula Ia (for example, the monoacetone solvate of the compound of Formula I), it is believed that the invention will find its best utility when integrated into a process for preparing a compound of Formula Ia as part of the workup procedure leading to isolation of the compound from the reaction mixture in the form of an acetone solvate

Without wanting to be bound by theory, it is believed that the present invention can be carried out as part of a workup process using reaction mixtures in which the imine of Formula Ic' is present to various levels as an unreacted impunity without adversely effecting the purity of the crystalline solvate provided by the present process, however, in some embodiments it is preferred to react the imine as closely as possible to completion, maximizing the utilization of the compound of Formula Ic', an expensive reagent with several chiral centers.

It will be appreciated that, if desired, recrystallization of the isolated purified mono-acetone solvate compound of Formula Ia from acetone can be further purified by recrystallization from acetone. Accordingly, the present process can be coupled with additional purification processes to further improve the purity of the material provided, but in most applications, the present invention process for precipitating the crystalline acetone solvate of the compound of Formula I from acetone workup of the reaction mixture provides suitably pure material to employ in the preparation of the compound of Formula II. In some embodiments the process of the invention is employed to provide impurity levels in the compound of Formula Ia of less than 3.0 mole%. In some embodiments the process of the invention is employed to provide impurity levels in the compound of Formula Ia of less than 2.0 mole%, the impurity levels attained depending upon the level of impurities in the reaction mixture being worked up, the temperatures regimes selected in which the process is run and the contract time between the purifying solvent and the solids obtained from the process. It will be appreciated that when other purification solvents are employed in the workup of the reaction mixture providing a compound of Formula I the precipitation product may also be further purified by other known means if desired

There follows examples which illustrate the inventive precipitation process and compare it to the product obtained when the product of Formula Ia is precipitated directly from an ethanol/water reaction medium.

### EXAMPLES

The reagents and solvents used in the examples are articles of commerce which, unless otherwise noted, were used as received. Completion of a reaction step is generally taken to be when 99% of the substrate used has been consumed, generally as determined by published chromatography methods.

### Example 1: Preparation of the Compound of Formula I

### Using An Acetone Precipitation Process

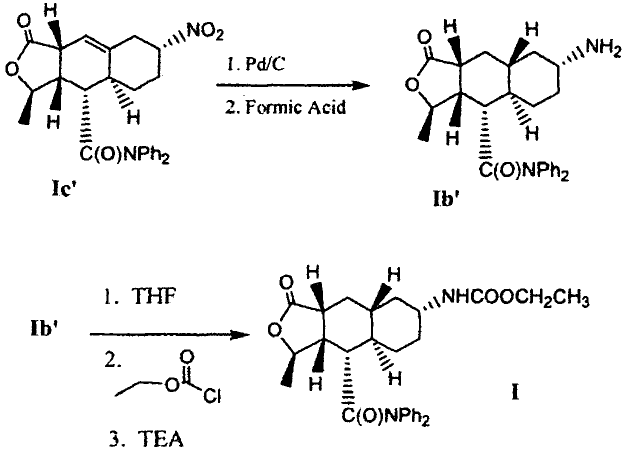

Into a three-neck flask equipped with an agitator, thermometer and nitrogen inlet were sequentially added the compound of Formula Ic' (100 g), THF (600 ml), 10% palladium on carbon (50% wet, 35g) and water (400 ml). The mixture was agitated for 10 minutes at room temperature and then heated to 50°C. Formic acid (70 mL) was added slowly while the temperature was maintained between 45 °C and 55°C. The reaction mixture was agitated for 7 hours while maintaining the temperature between 45 °C and 55 °C. After the reaction was judged complete by HPLC, the reaction mixture was cooled to 20°C and the pH was adjusted until the pH of the reaction mixture was between pH 1 and pH 2 by adding 60 mL of 25% sulfuric acid. After adjusting the pH of the reaction mixture, THF (200 mL) was added to the reaction mixture, and the mixture was filtered through a pad of Celite to remove the catalyst. The flask was rinsed with an aliquot of a mixture of THF (300 mL), water (300 mL) and 25% H₂SO₄ (1.5 mL). This rinse was passed throught the Celite and catalyst cake. The combined reaction mixture solution was charged back into a clean flask and the solution was cooled to below 10°C. The temperature of the solution was maintained at a temperature below 10 °C, and the pH of the solution was adjusted to pH 9 by adding 30 mL of 25% NaOH while maintaining the temperature. Following the pH adjustment, 150 g of NaCl was then added to the solution and it was warmed to 20°C and two phases were separated. The aqueous phase was extracted with 400 mL of THF and combined with the organic phase. The combined organics were washed with a brine solution (40 g of NaCl in 200 ml of water). The organic phase was cooled and maintained between 0°C and 10 °C, then methyl chloroformate (30 ml) was added to the combined organics. After addition of ethylchloroformate was complete, 56 mL of triethyl amine was slowly added to the reaction mixture while maintaining the temperature between 0-10°C. The mixture was stirred for 30 minutes while maintaining the temperature of the reaction mixture between 0 °C and 10°C. When the reaction was judged complete by HPLC, 200 ml of MTBE and 200 ml of water were added to the reaction mixture and the two phases were separated. To the organic phase was added another 200 ml water and 30 ml 5% sodium bicarbonate solution. The mixture was agitated for 1 hour maintaining the temperature of the mixture between 15 °C and 25°C. At the end of one hour of agitation, 25% aqueous sulfuric acid was added to the mixture until the pH of the mixture was between pH 6 and pH 7.5 and the two phases were separated. The separated organic layer was atmospherically concentrated to 300 mL and acetone (1000 mL) was added to the concentrated organic layer. The resulting solution was azeotropically distilled, to a volume of 300 mL. Additional acetone (800 mL) was added to the concentrated solution and the resulting solution was concentrated atmospherically to a volume of 450 mL The compound of Formula I was precipitated from the concentrated acetone solution by cooling the solution and maintaining the temperature between 45 °C and 60°C until a precipitate was observed (about 1 hour) followed by cooling the solution over three hours to a temperature of from -5°C to + 10 °C. The temperature of the resulting slurry was maintained between -5 °C and +10 °C and agitated for an additional 1-2 hours After a cold agitation period was complete, the precipitated solids were isolated by vacuum filtration. The filter cake was washed with 250 ml of acetone maintained at a temperature between -5°C and +10°C. The solid product was dried for 6 hours under vacuum at a temperature maintained between at 15°C and 25 °C to provide a monoacetone solvate form of the compound of Formula I as a white to off-white powder (90g, 84 mole% yield, 99.3 area% purity). MP 134-139°C, ¹H NMR (CDCl₃) δ 7.25 - 7.55 (m, 10 H), 4 89(m, 1H), 4.51 (bs, 1H), 4 09 (d, J = 6.98 Hz, 2H), 3.49 (brs, 1H), 2.41 (m, 2H) 2.25 (m, 1H), 2.19(s, H Acetone), 2.06 (d, J = 10.8 Hz, 2H), 1 96 (d, J = 10 9 Hz, 1H), 1.83 (ddd, J = 13 5, 6.09, 2.51 Hz, 1H), 1 63(m, 1H), 1.52 (d, J = 5.8 Hz, 3H), 1.23 (m, 5H), 1.17 (q, J = 11 5 Hz, 2H), 0 92 (q, J = 11.5 Hz, 1H)

### Example 1a: Preparation of the Compound of Formula I

### Using A 2-Methyl-Tetrahydrofuran Precipitation Process

The reaction outlined in Example 1 was carried out a second bme using a 2-methyl-tetrahydrofuran purification solvent to precipitate the compound of Formula I after workup of the reaction products. Thus, into a three-neck flash equipped with an agitator, thermometer and nitrogen inlet were sequentially added the compound of Formula Ic' (100g), THF (600 ml), 10% palladium on carbon (50% wet, 35g) and water (400 ml). The mixture was agitated for about 10 minutes at room temperature and then heated to 50°C Formic acid (70 ml) was added slowly while the temperature was maintained between 45°C and 55°C, then, while maintaining this temperature range, the reaction mixture was agitated for 4 hours. After the reaction was judged complete by HPLC, the reaction mixture was cooled to 20°C and the pH was adjusted to a pH between pH1 and pH 2 with the addition of 60 mL of 25% H₂SO₄. THF (200mL) was added to the reaction mixture, which was then filtered through a pad of Celite to remove the catalyst. A mixed solution of THF (300 mL), water (300 ml) and 25% H₂SO₄(1.5 mL) was used to rinse the flask and catalyst, and filtered through the Celite. The combined solution containing compound of Formula Ib' was charged back into a clean flask and the mixture was cooled to below 10°C. The pH was adjusted to pH 9 with the addition of 30 mL of 25% aqueous NaOH (30 mL) while maintaining the temperature of the reaction mixture below 10°C.

Following the addition of NaOH, and while continuing to maintain the temperature of the reaction mixture, 150 g of NaCl was added. The reaction mixture was warmed to 20°C and two phases were separated. The aqueous phase was extracted with THF (400 mL). The extract and organic phase were combined and washed with a brine solution (40 g of NaCl in 200 mL of water).

The organic phase was separated and cooled, and maintained at a temperature of from 0 °C to 10°C. Into the cooled organic phase was added ethyl chloroformate (30 ml). The mixture was maintained at a temperature of from 0 °C to 10°C while 56 mL of triethyl amine was slowly added to it. When addition of the ethyl chloroformate was complete, the mixture was stirred while maintaining the temperature. After the reaction was judged complete by HPLC, 200 ml of MTBE and 100 mL of water were added to the reaction mixture, followed by the slow addition of 100 mL of 25% H₂SO₄. The resulting organic phase was separated and concentrated to a volume of 300 mL by distilling off volatiles from the organic phase at a temperature between 70 °C and 80°C. To the concentrate was added 1000 ml of 2-methyl-tetrahydrofuran and the mixture was concentrated by atmospheric distillation to a volume of 300 mL. To this concentrate was added 800 mL of 2-methyl-tetrahydrofuran and the resulting mixture was concentrated atmospherically to a volume of 500 ml. The compound of Formula I was precipitated from the concentrated 2-methyl-tetrahydrofuran solution by cooling the solution and maintaining the temperature between 45 °C and 60°C until a precipitate was observed (about 1 hour) followed by cooling the solution over three hours to a temperature of from -5 °C to +10 °C. The temperature of the resulting slurry was maintained between -5 °C and +10 °C and agitated for an additional 1-2 hours. After a cold agitation period was complete, the precipitated solids were isolated by vacuum filtration. The filter cake was washed with 200 ml 2-methyl-tetrahydrofuran of maintained at a temperature between -5°C and + 10°C. The isolated solid product was dried for 12 hours in an air draft oven at a temperature maintained at 70 °C to provide the compound of Formula I as a mono-2-methyl-tetrahydrofuran solvate white to off-white powder (90g, 80 mole % yield, 98.4% pure by HPLC area% purity); ¹HNMR (CDCl₃) δ 7.25 - 7.55 (m, 10 H), 4.89(m, 1H), 4.51 (bs, 1H), 4.09 (d, J = 6.98 Hz, 2H), 3 98-3.86 (m, 2H. 2-Me-THF), 3.73-3.68 (m, 1H, 2-Me-THF), 3.49 (brs, 1H), 2.41 (m, 2H), 2 25 (m, 1H), 2.06 (d, J = 10.8 Hz, 2H), 1.96 (d, J = 10.9 Hz, 1H), 1.92-1.79(m, 3H, 2-Me-THF), 1.83 (ddd, J = 13.5, 6.09, 2.51 Hz, 1H), 1.63(m, 1H), 1 52 (d, J = 5.8 Hz, 3H), 1.45-1.36(m, 1H, 2-methyl-THF) 1.23 (m, 5H), 1 22 (d, 3H, 2-Me-THF) 1.17 (q, J =11.5 Hz, 2H), 0.92 (q, J = 11.5 Hz, 1H).

### Example 2: Preparation of the Compound of Formula I

### Using an Ethanol/Water Precipitation Process

The redaction outlined in Example 1 was carried out a third time using an ethanol/water purification solvent to precipitate the compound of Formula I after workup of the reaction products. Thus, into a three-neck flask equipped with an agitator, thermometer and nitrogen inlet were sequentially added the compound of Formula Ic' (100g), THF (600 ml), 10% palladium on carbon (50% wet, 35g) and water (400 ml). The mixture was agitated for about 10 minutes at room temperature and then heated to 50°C Formic acid (70 ml) was added slowly while the temperature was maintained between 45°C and 55°C, then, while maintaining this temperature range, the reaction mixture was agitated for 4 hours. After the reaction was judged complete by HPLC, the reaction mixture was cooled to 20°C and the pH was adjusted to a pH between pH1 and pH 2 with the addition of 60 mL of 25% H₂SO₄. THF (200mL) was added to the reaction mixture, which was then filtered through a pad of Celite to remove the catalyst. A mixed solution of THF (300 mL), water (300 ml) and 25% H₂SO₄(1.5 mL) was used to rinse the flask and catalyst, and filtered through the Celite. The combined solution containing compound of Formula Ib' was charged back into a clean flask and the mixture was cooled to below 10°C. The pH was adjusted to pH 9 with the addition of 30 mL of 25% aqueous NaOH (30 mL) while maintaining the temperature of the reaction mixture below 10°C. While maintaining the temperature of the reaction mixture, 150 g of NaCl was added. The reaction mixture was warmed to 20°C and two phases were separated. The aqueous phase was extracted with THF (400 mL). The extract and organic phase were combined and washed with a brine solution (40 g of NaCl in 200 mL of water). The resulting organic phase was separated and cooled to 5°C. While maintaining the temperature of the organic phase, 56 mL of triethyl amine was added to the cold organic phase followed by slow addition of 23.6 mL of ethyl chloroformate. When addition of the ethyl chloroformate was complete, the mixture was warmed to 20°C and stirred for 30 minutes. After the reaction was judged complete by HPLC, 200 ml of MTBE and 100 mL of water were added to the reaction mixture, followed by the slow addition of 100 mL of 25% H₂SO₄. The resulting organic phase was separated and concentrated to a volume of 300 mL by distilling off volatiles from the organic phase at a temperature between 70 °C and 80°C. To the concentrate was added 1000 ml of ethanol, and the mixture was concentrated by atmospheric distillation to a volume of 300 mL. To the concentrate was added another 800 mL of ethanol and atmospheric distillation was repeated to provide a concentrate of 300 mL volume. In general, addition of ethanol with subsequent concentration by distillation is repeated until THF content is observed to be below 5% normalized area by GC.

The temperature of the concentrate was maintained between 60 °C and 85 °C, and water (250 mL) was added to the concentrate. The mixture was cooled to a temperature of between 55 °C and 65 °C and the compound of Formula I was precipitated from the mixture with seeding. After agitating for 1 hour and maintaining the temperature of the concentrate at a temperature between 55 °C and 65 °C, 150 ml water was added at this temperature and the mixture was held at a temperature between 55 °C and 65 °C for 1 hour. The mixture was then cooled and maintained at a temperature between 15 °C and 25°C, with agitation for an additional 3 hours and then the product was separated from the concentrate by vacuum filtration and washed with ethanol-water. The product was dried at 50-60°C to provide an off-white solid (86g, Yield: 85%), Mp 188.2°C ¹HNMR (CDCl₃) δ 7.25 - 7.55 (m, 10 H), 4.89(m, 1H), 4.51 (bs, 1H), 4.09 (d, J = 6.98 Hz, 2H), 3.49 (brs, 1H), 2.41 (m, 2H), 2.25 (m, 1H), 2.06 (d, J = 10.8 Hz, 2H), 1.96 (d, J = 10.9 Hz, 1H), 1.83 (ddd, J = 13.5, 6.09, 2.51 Hz, 1H), 1.63(m, 1H), 1.52 (d, J = 5.8 Hz, 3H), 1.23 (m, 5H), 1.17 (q, J = 11.5 Hz, 2H), 0.92 (q, J = 11.5 Hz, 1H). MS (ESI) for M+H calcd. 491 Found: 491.

### Example 3: Comparison of Impurity Levels in Compound Prepared

### In Accordance With the Process of Examples 1 and 2

The procedures described in Examples 1 and 1a for the preparation of the monoacetone solvate and mono-2-methyl-tetrahydrofuran solvate respectively of the compound of Formula I and in Example 2 for the preparation of crystalline material precipitated from ethanol/water were repeated. For each of the repeated procedures, the level of impurities present in a sample of each procedure were evaluated by HPLC methods. The results are presented below in Table 1.

**Table 1**

| | | | |
|---|---|---|---|
| Impurity Level Observed in Sample (Product / total of detected impurities)* | | | |

| Sample No. | Process of Example 1 | Process of Example 1a | Process of Example 2 |
|---|---|---|---|
| 1 | 99.29/0.71 | 98.4/1.60 | 87.11/10.67 |
| 2 | 99.39/0.61 | 98.2/1.80 | 82.64/14.74 |
| 3 | 99.18/0.82 | 98.3/1.70 | 87.22/10.41 |

| | | | |
|---|---|---|---|
| *Determined by HPLC of sample of isolated precipitate, expressed as area of HPLC peak associated with precipitated product/total of HPLC peak areas associated with detectable impurities present in sample normalized to 100%. | | | |

These data show that the process of the present invention provides about a reduction of about a factor of 10 in the impurity level present in the 2-methyl-tetrahydrofuran solvate and well in excess of a factor of 10 reduction in the total amount of impurities present in the acetone solvate precipitation product These data show also that the process of the present invention, while providing improvements in the level of impurities present in the precipitate, maintains or exceeds the yields of the compound of Formula I obtained from the prior process.

## Claims

1. A process for providing a purified, precipitated form of the compound of Formula I, 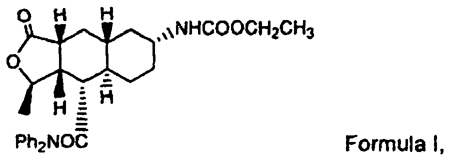 the process comprising:
a. providing a saturated solution comprising the compound of Formula I in a solvent comprising substantially acetone or 2-methyl-tetrahydrofuran (2-Me-THF), at a first temperature, wherein the first temperature is above ambient and is selected to be in a range suitable to form a suspension comprising crystals of the compound of Formula I and maintaining the solution at said first temperature for a period of time sufficient to form a suspension of the compound of Formula I, optionally while agitating the mixture;
b. optionally reducing the temperature of the suspension formed in Step "a" to a second temperature, wherein said second temperature is subambient;
c. optionally agitating the suspension from step "b" while holding it at said second temperature for a period sufficient to precipitate a crystalline product having less than 3.0 mole% impurity and comprising at least 80% of the compound of Formula I initially present in the solution provided in Step "a"; and
d. optionally isolating the crystalline product precipitated in Step "c".

2. The process of claim 1 wherein, when step "c" is carried out it is carried out at a second temperature and for a period sufficient to precipitate a crystalline product having less than 2.0 mole% impurity.

3. The process of claim 2 wherein, said first temperature is a temperature of from 45 °C to 60 °C.

4. The process of claim 3 wherein said second temperature is a temperature of from -5°C to +10°C.

5. The process of claim 4 wherein the solution in Step "a" is maintained at said first temperature for 1 hour.

6. The process of claim 5 wherein the period of time over which the temperature is reduced from said first temperature to said second temperature in Step "b" is a period of 3 hours.

7. The process of claim 6 wherein, the solution in Step "a" is provided by adding a purification solvent which is acetone or 2-methyl-tetrahydrofuran to a solution comprising: the compound of Formula I; methyl tertiarybutylether; and tetrahydrofuran, and distilling off volatiles until the solvent remaining is substantially the purification solvent

8. A process for synthesis of the compound of Formula la 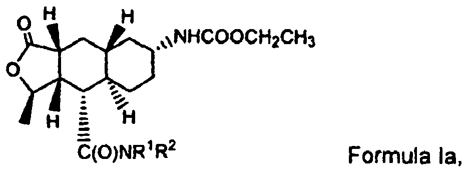 the process comprising:
(a) providing a THF solution of the compound of Formula Ib 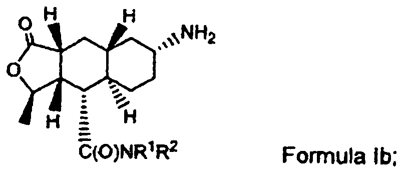
(b) forming the amide of Formula Ia in a reaction mixture provided by treating the THF solution from Step "a" first with ethylchloroformate and adding thereafter triethyl amine;
(c) working up the reaction mixture from Step "b" by adding equal volumes of water and methyl tert-butyl ether (MTBE) and then separating the resulting organic and aqueous phases;
(d) mixing the separated organic phase from Step "c" with an equal volume of water and an aliquot of a carbonate base solution;
(e) adjusting the pH of the mixture provided in Step "d" with an aqueous acid to a pH of from pH 6 to pH 7.5 and separating the aqueous and organic portions of the mixture;
(f) modifying the solvent in the separated organic phase obtained in Step "e" by a process comprising:
(i) mixing a purifying solvent with the separated organic phase obtained in Step "e", wherein the purifying solvent is acetone or 2-Me-THF;
(ii) azetropically distilling the mixture from step "f(i)" until it is concentrated; and
(iii)repeating steps "f(i)" and "f(ii)" until the solvent in the mixture of step "f(ii)" comprises substantially the purifying solvent and
(g) cooling the phase provided in Step "f(iii)", thereby precipitating a substantially pure solvate form of the compound of Formula Ia.

9. The process of claim 8 wherein the compound of Formula Ib is provided by hydrogenating the compound of Formula Ic, 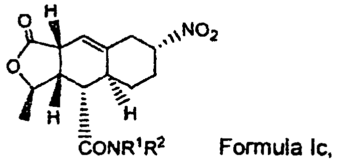 using a palladium catalyst and a mixed water/THF reaction medium.

10. The process of claim 9, wherein R¹ and R² in each occurrence is phenyl, and therefore the compound of Formula Ia formed is the compound of Formula I,

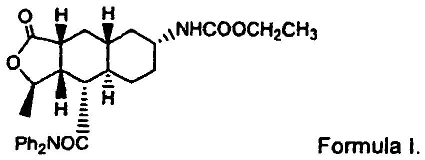

11. The process of claim 10 wherein, in Step "d", the carbonate base solution is an aqueous 5% sodium bicarbonate solution.

12. The process of claim 11 wherein, in Step "e", the aqueous acid used to adjust the solution pH is an aqueous sulfuric acid solution.

13. The process of claim 12 wherein, said separated organic portion formed in step "e" is concentrated by distilling off solvent prior to adding the purifying solvent in Step "f".

14. The process of claim 13 wherein Step "g", the cooling step, comprises: (i) adjusting the temperature of said modified organic phase prepared in step "f" to a first temperature, wherein the first temperature is a temperature above ambient and is selected to permit formation of a suspension comprising the compound of Formula Ia, and maintaining said modified organic phase at said first temperature for a period sufficiently long to form a suspension comprising the compound of Formula Ia; and (ii) adjusting the temperature of the suspension formed in step "i" to a second temperature, wherein said second temperature is subambient, and holding the suspension at that temperature for a period sufficient to produce a crystalline product having less than 2.0 mole% impurity and comprising at least 80% of the compound of Formula Ia initially present in said modified organic phase prepared in step "f".

15. The process of claim 14 wherein, said first temperature is a temperature of from 45 °C to 60 °C.

16. The process of claim 15 wherein, during step "g(i)", the suspension is stirred.

17. The process of claim 16 which further comprises isolating the precipitated solids from step "g(ii)".

18. The process of claim 16, wherein said second temperature is from (-5 °C) to (+10) °C.

19. The process of claim 18 wherein, the temperature of the suspension is adjusted in step "g(ii)" from said first temperature to said second temperature over a period of three hours.

## Patentansprüche

1. Ein Verfahren zur Bereitstellung einer gereinigten gefällten Form der Verbindung der Formel I, 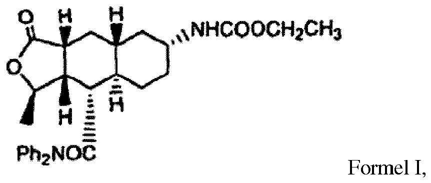 wobei das Verfahren umfasst:
a. Bereitstellen einer gesättigten Lösung, umfassend die Verbindung der Formel I in einem Lösungsmittel, das im Wesentlichen Aceton oder 2-Methyltetrahydrofuran (2-Me-THF) umfasst, bei einer ersten Temperatur, wobei die erste Temperatur über der Umgebungstemperatur liegt und so ausgewählt ist, dass sie in einem Bereich liegt, der zur Bildung einer Suspension geeignet ist, welche Kristalle der Verbindung der Formel I umfasst, und Halten der Lösung bei der ersten Temperatur für einen ausreichenden Zeitraum, um eine Suspension der Verbindung der Formel I zu bilden, während die Mischung gegebenenfalls gerührt wird,
b. gegebenenfalls Verringern der Temperatur der in Schritt "a" gebildeten Suspension auf eine zweite Temperatur, wobei die zweite Temperatur unter der Umgebungstemperatur liegt,
c. gegebenenfalls Rühren der Suspension von Schritt "b", während sie bei der zweiten Temperatur gehalten wird, für einen ausreichenden Zeitraum, um ein kristallines Produkt auszufällen, das weniger als 3,0 Mol-% Verunreinigungen besitzt und wenigstens 80% der Verbindung der Formel I, die ursprünglich in der in Schritt "a" bereitgestellten Lösung vorhanden war, umfasst, und
d. gegebenenfalls Isolieren des in Schritt "c" ausgefällten kristallinen Produkts.

2. Das Verfahren nach Anspruch 1, wobei, wenn Schritt "c" durchgeführt wird, er bei einer zweiten Temperatur und für einen ausreichenden Zeitraum durchgeführt wird, um ein kristallines Produkt mit weniger als 2,0 Mol-% Verunreinigungen auszufällen.

3. Das Verfahren nach Anspruch 2, wobei die erste Temperatur eine Temperatur von 45°C bis 60°C ist.

4. Das Verfahren nach Anspruch 3, wobei die zweite Temperatur eine Temperatur von -5°C bis +10°C ist.

5. Das Verfahren nach Anspruch 4, wobei die Lösung in Schritt "a" 1 Stunde bei der ersten Temperatur gehalten wird.

6. Das Verfahren nach Anspruch 5, wobei der Zeitraum, innerhalb dessen die Temperatur von der ersten Temperatur auf die zweite Temperatur in Schritt "b" verringert wird, ein Zeitraum von 3 Stunden ist.

7. Das Verfahren nach Anspruch 6, wobei die Lösung in Schritt "a" durch Zugabe eines Reinigungslösungsmittels, welches Aceton oder 2-Methyltetrahydrofuran ist, zu einer Lösung, umfassend: die Verbindung der Formel I, Methyl-tert-butylether und Tetrahydrofuran, und Abdestillieren der flüchtigen Bestandteile, bis das verbleibende Lösungsmittel im Wesentlichen das Reinigungslösungsmittel ist, bereitgestellt wird.

8. Ein Verfahren zur Synthese der Verbindung der Formel Ia 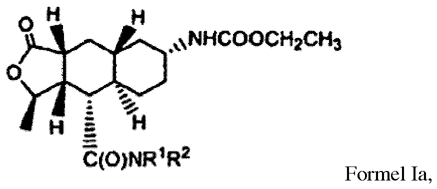 wobei das Verfahren umfasst:
(a) Bereitstellen einer THF-Lösung der Verbindung der Formel Ib 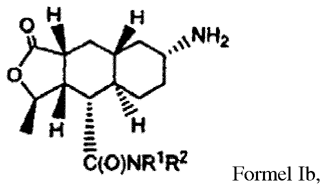
(b) Bilden des Amids der Formel Ia in einer Reaktionsmischung, die durch Behandeln der THF-Lösung von Schritt "a" zunächst mit Ethylchlorformiat und anschließende Zugabe von Triethylamin bereitgestellt wird,
(c) Aufarbeiten der Reaktionsmischung von Schritt "b" durch Zugabe gleicher Volumen Wasser und Methyl-tert-butylether (MTBE) und anschließendes Trennen der resultierenden organischen und wässrigen Phasen,
(d) Vermischen der abgetrennten organischen Phase von Schritt "c" mit einem gleichen Volumen Wasser und einem Aliquot einer Carbonatbasenlösung,
(e) Einstellen des pH-Werts der in Schritt "d" bereitgestellten Mischung mit einer wässrigen Säure auf einen pH-Wert von pH 6 bis pH 7,5 und Trennen der wässrigen und organischen Teile der Mischung,
(f) Modifizieren des Lösungsmittels in der in Schritt "e" erhaltenen abgetrennten organischen Phase durch ein Verfahren, umfassend:
(i) Vermischen eines Reinigungslösungsmittels mit der in Schritt "e" erhaltenen abgetrennten organischen Phase, wobei das Reinigungslösungsmittel Aceton oder 2-Me-THF ist,
(ii) azeotropes Destillieren der Mischung von Schritt "f(i)", bis diese aufkonzentriert ist, und
(iii) Wiederholen der Schritte "f(i)" und "f(ii)", bis das Lösungsmittel in der Mischung von Schritt "f(ii)" im Wesentlichen das Reinigungslösungsmittel umfasst, und
(g) Abkühlen der in Schritt "f(iii)" bereitgestellten Phase, wodurch eine im Wesentlichen reine Solvatform der Verbindung der Formel Ia ausgefällt wird.

9. Das Verfahren nach Anspruch 8, wobei die Verbindung der Formel Ib durch Hydrieren der Verbindung der Formel Ic, 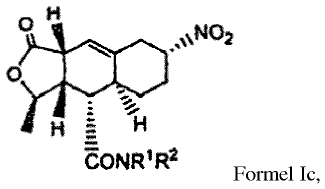 unter Verwendung eines Palladiumkatalysators und eines gemischten Wasser/THF-Reaktionsmediums bereitgestellt wird.

10. Das Verfahren nach Anspruch 9, wobei R¹ und R² bei jedem Auftreten Phenyl sind und daher die gebildete Verbindung der Formel Ia die Verbindung der Formel I ist

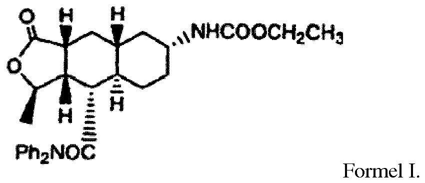

11. Das Verfahren nach Anspruch 10, wobei in Schritt "d" die Carbonatbasenlösung eine wässrige 5%ige Natriumhydrogencarbonatlösung ist.

12. Das Verfahren nach Anspruch 11, wobei in Schritt "e" die wässrige Säure, die zum Einstellen des pH-Werts der Lösung verwendet wird, eine wässrige Schwefelsäurelösung ist.

13. Das Verfahren nach Anspruch 12, wobei der in Schritt "e" gebildete abgetrennte organische Teil durch Abdestillieren des Lösungsmittels vor der Zugabe des Reinigungslösungsmittels in Schritt "f" aufkonzentriert wird.

14. Das Verfahren nach Anspruch 13, wobei Schritt "g", der Abkühlschritt, umfasst: (i) das Einstellen der Temperatur der in Schritt "f" hergestellten modifizierten organischen Phase auf eine erste Temperatur, wobei die erste Temperatur eine Temperatur über Umgebungstemperatur ist und so ausgewählt ist aus, dass die Bildung einer Suspension ermöglicht wird, welche die Verbindung der Formel Ia umfasst, und das Halten der modifizierten organischen Phase bei der ersten Temperatur für einen Zeitraum, der ausreichend lang ist, um eine Suspension zu bilden, die die Verbindung der Formel Ia umfasst, und (ii) das Einstellen der Temperatur der in Schritt "i" gebildeten Suspension auf eine zweite Temperatur, wobei die zweite Temperatur unter Umgebungstemperatur liegt, und das Halten der Suspension bei dieser Temperatur für einen ausreichenden Zeitraum, um ein kristallines Produkt zu erzeugen, das weniger als 2,0 Mol-% Verunreinigungen besitzt und wenigstens 80% der Verbindung der Formel Ia, die ursprünglich in der in Schritt "f" hergestellten modifizierten organischen Phasen vorhanden war, umfasst.

15. Das Verfahren nach Anspruch 14, wobei die erste Temperatur eine Temperatur von 45°C bis 60°C ist.

16. Das Verfahren nach Anspruch 15, wobei während Schritt "g(i)" die Suspension gerührt wird.

17. Das Verfahren nach Anspruch 16, das ferner das Isolieren der ausgefällten Feststoffe von Schritt "g(ii)" umfasst.

18. Das Verfahren nach Anspruch 16, wobei die zweite Temperatur (-5°C) bis (+10)°C beträgt.

19. Das Verfahren nach Anspruch 18, wobei die Temperatur der Suspension in Schritt "g(ii)" über einen Zeitraum von drei Stunden von der ersten Temperatur auf die zweite Temperatur eingestellt wird.

## Revendications

1. Procédé pour la fourniture d'une forme précipitée, purifiée du composé de la Formule I: 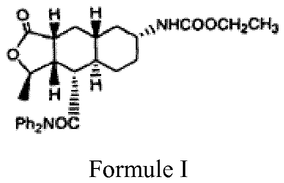 le procédé comprenant:
a. la fourniture d'une solution saturée comprenant le composé de la Formule I dans un solvant comprenant substantiellement de l'acétone ou du 2-méthyl-tétrahydrofurane (2-Me-THF), à une première température, dans lequel la première température est au-dessus de l'ambiante et elle est choisie pour être dans un intervalle approprié pour former une suspension comprenant des cristaux du composé de la Formule I et le maintien de la solution à ladite première température pendant une période de temps suffisante pour former une suspension du composé de la Formule I, éventuellement tout en agitant le mélange;
b. éventuellement la réduction de la température de la suspension formée dans l'étape « a » à une deuxième température, dans lequel ladite deuxième température est sous l'ambiante;
c. éventuellement l'agitation de la suspension provenant de l'étape « b » tout en la maintenant à ladite deuxième température pendant une période suffisante pour précipiter un produit cristallin possédant moins de 3,0% en moles d'impureté et comprenant au moins 80% du composé de la Formule I initialement présent dans la solution fournie dans l'étape « a »; et
d. éventuellement l'isolement du produit cristallin précipité dans l'étape « c ».

2. Procédé selon la revendication 1, dans lequel, lorsque l'étape « c » est réalisée, elle est réalisée à une deuxième température et pendant une période suffisante pour précipiter un produit cristallin possédant moins de 2,0% en moles d'impureté.

3. Procédé selon la revendication 2, dans lequel ladite première température est une température de 45°C à 60°C.

4. Procédé selon la revendication 3, dans lequel ladite deuxième température est une température de -5°C à +10°C.

5. Procédé selon la revendication 4, dans lequel la solution dans l'étape « a » est maintenue à ladite première température pendant 1 heure.

6. Procédé selon la revendication 5, dans lequel la période de temps pendant laquelle la température est réduite de ladite première température à ladite deuxième température dans l'étape « b » est une période de 3 heures.

7. Procédé selon la revendication 6, dans lequel la solution dans l'étape « a » est fournie en ajoutant un solvant de purification qui est l'acétone ou le 2-méthyl-tétrahydrofurane à une solution comprenant: le composé de la Formule I; du tert-butyléther de méthyle; et du tétrahydrofurane, et en séparant par distillation les produits volatils jusqu'à ce que le solvant restant soit substantiellement le solvant de purification.

8. Procédé pour la synthèse du composé de la Formule Ia: 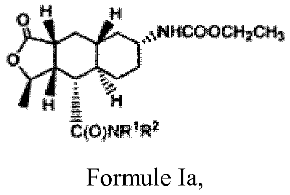 le procédé comprenant:
(a) la fourniture d'une solution de THF du composé de la Formule Ib: 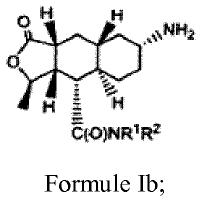
(b) la formation de l'amide de la Formule Ia dans un mélange de réaction obtenu par le traitement de la solution de THF provenant de l'étape « a » tout d'abord avec de l'éthylchloroformate et l'ajout par la suite de triéthylamine;
(c) le travail du mélange de réaction provenant de l'étape « b » en ajoutant des volumes égaux d'eau et de tert-butyléther de méthyle (MTBE) et ensuite en séparant les phases organique et aqueuse résultantes;
(d) le mélange de la phase organique séparée provenant de l'étape « c » avec un volume égal d'eau et d'un aliquote d'une solution de base de carbonate;
(e) l'ajustement du pH du mélange obtenu dans l'étape « d » avec un acide aqueux à un pH de pH 6 à pH 7,5 et la séparation des portions aqueuse et organique du mélange;
(f) la modification du solvant dans la phase organique séparée obtenue dans l'étape « e » par un procédé comprenant:
(i) le mélange d'un solvant de purification avec la phase organique séparée obtenue dans l'étape « e », où le solvant de purification est l'acétone ou 2-Me-THF;
(ii) la distillation azéotrope du mélange provenant de l'étape « f(i) » jusqu'à ce qu'il soit concentré; et
(iii) la répétition des étapes « f(i) » et « f(ii) » jusqu'à ce que le solvant dans le mélange de l'étape « f(ii) » comprenne substantiellement le solvant de purification; et
(g) le refroidissement de la phase obtenue dans l'étape « f(iii) », précipitant ainsi une forme substantiellement pure de solvate du composé de la Formule Ia.

9. Procédé selon la revendication 8, dans lequel le composé de la Formule Ib est obtenu par l'hydrogénation du composé de la Formule Ic: 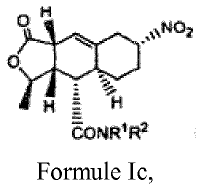 en utilisant un catalyseur de palladium et un milieu de réaction d'eau/THF mélangés.

10. Procédé selon la revendication 9, dans lequel R¹ et R² dans chaque occurrence sont un phényle et par conséquent le composé de la Formule Ia formé est le composé de la Formule I:

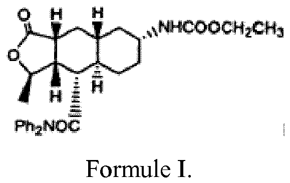

11. Procédé selon la revendication 10, dans lequel, dans l'étape « d », la solution de base de carbonate est une solution aqueuse de 5% de bicarbonate de sodium.

12. Procédé selon la revendication 11, dans lequel, dans l'étape « e », l'acide aqueux utilisé pour ajuster le pH de la solution est une solution aqueuse d'acide sulfurique.

13. Procédé selon la revendication 12, dans lequel ladite portion organique séparée formée dans l'étape « e » est concentrée en séparant par distillation le solvant avant l'ajout du solvant de purification dans l'étape « f ».

14. Procédé selon la revendication 13, dans lequel l'étape « g », l'étape de refroidissement, comprend:
(i) l'ajustement de la température de ladite phase organique modifiée préparée dans l'étape « f » à une première température, dans lequel la première température est une température au-dessus de l'ambiante et elle est choisie pour permettre la formation d'une suspension comprenant le composé de la Formule Ia, et le maintien de ladite phase organique modifiée à ladite première température pendant une période suffisamment longue pour former une suspension comprenant le composé de la Formule Ia; et (ii) l'ajustement de la température de la suspension formée dans l'étape « i » à une deuxième température, dans lequel ladite deuxième température est sous l'ambiante, et le maintien de la suspension à cette température pendant une période suffisante pour donner un produit cristallin possédant moins de 2,0% en moles d'impureté et comprenant au moins 80% du composé de la Formule Ia initialement présent dans ladite phase organique modifiée préparée dans l'étape « f ».

15. Procédé selon la revendication 14, dans lequel ladite première température est une température de 45°C à 60°C.

16. Procédé selon la revendication 15, dans lequel, durant l'étape « g(i) », la suspension est agitée.

17. Procédé selon la revendication 16, qui comprend en outre l'isolement des solides précipités provenant de l'étape « g(ii) ».

18. Procédé selon la revendication 16, dans lequel ladite deuxième température est de -5°C à +10°C.

19. Procédé selon la revendication 18, dans lequel la température de la suspension est ajustée dans l'étape « g(ii) » de ladite première température à ladite deuxième température sur une période de trois heures.
